# EUROPEAN PATENT SPECIFICATION

(11) **EP 1 691 926 B1**
(45) Date of publication and mention of the grant of the patent: **05.05.2010**
(21) Application number: 04812490.3
(22) Date of filing: 30.11.2004
(51) Int. Cl.: B01L 3/00, B01L 3/14, C12Q 1/42

(54) **PHOSPHATASE INHIBITOR SAMPLE COLLECTION SYSTEM**
PROBENAHMESYSTEM MIT PHOSPHATASEINHIBITOR
SYSTEME DE PRELEVEMENT A INHIBITEUR ANTI-PHOSPHATASE

(30) Priority: 08.12.2003 US 527790 P
(43) Date of publication of application: 23.08.2006
(60) Divisional application: 10155786.6
(73) Proprietor: Becton, Dickinson and Company, Franklin Lakes, NJ 07417-1880 (US)
(72) Inventor: HAYWOOD, Bruce, C., Franklin Lakes, NJ 07417 (US)
(74) Representative: von Kreisler Selting Werner
(86) International application number: PCT/US2004/039969
(87) International publication number: WO 2005/056187

(56) References cited:
- WO-A2-03/097237
- US-A- 5 998 216
- US-A1- 2003 161 893

## Description

### FIELD OF THE INVENTION

The present invention is directed to a device for collecting and stabilizing a biological sample, particularly a whole blood sample, directly from a patient. More specifically, the present invention relates to sample collection containers having a stabilizing additive contained therein for stabilizing proteins immediately on collection of a biological sample and for inhibiting protein modifications during storage thereof.

### BACKGROUND OF THE INVENTION

The study of proteomics has recently increased significantly. Proteomics may encompass many meanings, but it involves looking at proteins, whether individually or, more typically, as patterns. For example, researchers are interested in the protein profiles that may be reflective of certain disease states, e.g., the profile of a healthy individual vs. the profile of a diseased individual may show differences that can be used as future indicators of disease states. As known in the art, mass spectrometry is a key tool used to look at such profiles. One challenge in such protein study is the many modifications a protein goes through in its lifetime, which are broadly called post-translational modifications. Given that the state of a protein changes over time, it is difficult to ensure that a profile of an individual will be consistent over time. Thus, a profile believed to be indicative of a disease state may only be valid for specific conditions, and thus not repeatable on a basis sufficient to serve as a diagnostic tool. Thus, devices and/or processes capable of addressing this variability would be desirable.

### SUMMARY OF THE INVENTION

The most common mechanism of communication between cells involves the release of signaling molecules, such as hormones, neurotransmitters, and growth factors, from one cell type that interact with and activate specific receptor proteins on the surface of target cells. The activated receptor then generates an intracellular signal that ultimately couples to specific functional processes in cells to produce a physiological response. Studies of the signal transduction pathways that couple receptor activation to these physiological responses represent one of the most active and important research areas in modem biology. Signal transduction studies are fundamental to disease research, drug discovery and development, and diagnostics. The reversible attachment of phosphate to serine, threonine and tyrosine residues of cellular proteins is a control mechanism that plays a key role in most if not all signal transduction pathways. Two types of enzymes control the extent and direction of phosphorylation of a particular cell protein:
Protein kinases add phosphate to the proteins (phosphorylation)
Protein phosphatases remove the phosphate. (dephosphorylation)
   These pathway effects continue to be active after biological samples are collected. Without understanding of these variable ex-vivo modifications, phosphate removal, in particular, can confound or impair research results. Protein phosphatases are classified based on their substrate specificity, dependence on metal ions, and sensitivity to inhibitory agents. A class of chemicals, protein phosphatase inhibitors, is commonly used to limit removal of phosphate groups. (Protein phosphates inhibitors are also used to treat diseases).

There are hundreds of inhibitors available through chemical suppliers and some even provide inhibitor cocktails with anywhere from two to five inhibitors pre-mixed. It is unfortunate, that by the time most of these inhibitors are applied, that the much of the activity being studied is "unnatural" or ex vivo artifact. For certain studies, it is important to be able to understand the state of cells in a manner that is closely representative of the *in vivo* physiology. For this reason, there is value in regulating dephosphorylation as close to "time zero" of specimen excision or extraction as possible.

The invention includes a diverse range of collection devices that contain one or more pre-loaded protein phosphatase inhibitors, such that when the specimen contacts the collection device it immediately comes in contact with the inhibitor and the dephosphorylation activity is regulated.

### BRIEF DESCRIPTION OF THE DRAWINGS

FIG. 1 is a perspective view of a typical blood collection tube.

FIG. 2 is a perspective view of a test plate.

FIG. 3a is a perspective view of a sample collection assembly, while FIG. 3b is a sectional view of the sample collection assembly.

FIG. 4 is a longitudinal sectional view of a syringe.

FIG. 5 is a longitudinal sectional view of another embodiment of a syringe.

FIG. 6a is a side view of a catheter assembly, while FIG. 6b is a partial side view of the catheter.

FIG. 7 is a perspective view of a pipette.

FIG. 8 is a perspective view illustrating a blood collecting bag.

### DETAILED DESCRIPTION OF THE INVENTION

While this invention is satisfied by embodiments in many different forms, there will herein be described in detail preferred embodiments of the invention, with the understanding that the present disclosure is to be considered as exemplary of the principles of the invention and is not intended to limit the invention to the embodiments illustrated and described. Numerous variations may be made by persons skilled in the art without departure from the spirit of the invention. The scope of the invention will be measured by the appended claims.

The present invention is directed to devices for stabilizing proteins in a biological sample to better enable downstream analysis. More particularly, the present invention is directed to devices for inhibiting the phosphorylation cascade in a biological sample during storage. According to the present invention, the device comprises a container containing an amount of a stabilizing agent comprising a phosphatase inhibitor for mixing with a biological sample immediately on collection of the sample.

Although it is possible to use the present invention with any protein-containing biological sample, preferably the biological sample is any body fluid withdrawn from a patient. Most preferably, the biological sample is whole blood or a component thereof. Examples of other biological samples include cell-containing compositions such as red blood cell concentrates, platelet concentrates, leukocyte concentrates, plasma, serum, urine, bone marrow aspirates, cerebral spinal fluid, tissue, cells, feces, saliva and oral secretions, nasal secretions, lymphatic fluid and the like.

The sample collection system of the present invention can encompass any collection device including, but not limited to, tubes such as test tubes and centrifuge tubes; closed system blood collection devices, such as collection bags; syringes, especially pre-filled syringes; catheters, such as central lines; microtiter and other multi-well plates; arrays; tubing; laboratory vessels such as flasks, spinner flasks, roller bottles, vials, microscope slides, microscope slide assemblies, coverslips, films and porous substrates and assemblies; pipettes and pipette tips, etc.; tissue and other biological sample collection containers; and any other container suitable for holding a biological sample, as well as containers and elements involved in transferring samples. In one aspect of the invention, a sample collection tube having a separating member (*e*.*g*., a mechanical separating element, a gel or a filter mechanism) for separating blood components is used. In such aspect, the interior of the tube and/or the exterior of the separating member may be treated with the stabilizing agent. According to the present invention, the collection device contains a stabilizing agent for stabilizing the biological sample.

Plastic or glass is often used to manufacture the collection device used in the present invention. Some preferred materials used to manufacture the collection device include polypropylene, polyethylene, polyethyleneterephthalate, polystyrene, polycarbonate and cellulosics. More expensive plastics such as polytetrafluoroethylene and other fluorinated polymers may also be used. In addition to the materials mentioned above, examples of other suitable materials for the collection devices used in the present invention include polyolefins, polyamides, polyesters, silicones, polyurethanes, epoxies, acrylics, polyacrylates, polysulfones, polymethacrylates, PEEK, polyimide and fluoropolymers such as PTFE Teflon®, FEP Teflon®, Tefzel®, poly(vinylidene fluoride), PVDF and perfluoroalkoxy resins. Glass products including silica glass are also used to manufacture the collection devices. One exemplary glass product is PYREX® (available from Coming Glass, Corning, New York). Ceramic collection devices can be used according to embodiments of the invention. Cellulosic products such as paper and reinforced paper containers can also be used to form collection devices according to the invention.

The stabilizing agent of the invention comprises one or more phosphatase inhibitors able to inhibit phosphorylation activity and the associated modification or, destruction of proteins during storage of a biological sample. The agent stabilizes the biological sample, such as a blood sample, to produce a stable composition that inhibits or prevents modification, degradation and/or fragmentation of proteins present in the biological sample. In accordance with one embodiment of the present invention, the collection device is pretreated with the stabilizing agent, preferably by the manufacturer, and is packaged in a ready-to-use form. Typically, the packaged collection device is sterile and is also packaged in sterile packaging materials.

The present invention could be used by pharmaceutical companies, biotechnology companies, contract research organizations, university researchers, research hospitals and any institution and individual who is interested in studying proteins. The present invention would enable researchers to conveniently and readily protect and process protein samples for downstream analysis. The collection device according to the present invention would serve as a front-end sample collection device aiding analytical objectives including, but not limited to the following: protein banking, protein identification and characterization, protein expression, protein quantitation, protein-protein interactions, development of protein function assays, protein target finding and validation, predictive toxicology, determination of drug action, drug validation, 3-D protein structural analysis and computer modeling. Clinical uses are also contemplated.

Preferably, the stabilizing agent comprises or consists of at least one phosphatase inhibitor. Suitable examples include inhibitors of serine or threonine phosphatases (e.g., the PPP or PPM families) and/or tyrosine phosphatases (the PTP family). For example, inhibitors of PP1 phosphatase include calyculin A, nodularin, NIPP-1, microcystin LR, tautomycin, okadaic acid, and cantharidin. Inhibitors of PP2A include calyculin A, microcystin LR, okadaic acid, fostriecin, tautomycin, cantharidin, endothall, and nodularin. Inhibitors of PP2B include cyclosporin A, FK 506/immunophilin complexes, cypermethrin, deltamethrin, and fenvalerate. Inhibitors of PTP include bpV(phen), dephostatin, mpV(pic) DMHV, and sodium orthovanadate. Phosphatases and inhibitors therefore are known in the art, and are commercially available, e.g., from Calbiochem of San Diego, California, USA.

Combinations of phosphatase inhibitors, commonly referred to as "cocktails" by commercial suppliers of such inhibitors, may also be used as the stabilizing agent. Such "cocktails" are generally advantageous in that they provide stabilization for a range of proteins of interest; therefore, a stabilizing agent containing more than two phosphatase inhibitors is generally desirable.

In addition, it may be desirable to include protease inhibitors with the phosphatase inhibitor, to further promote protein stability. Examples include inhibitors of proteases such as serine proteases, cysteine proteases, aspartic proteases, metalloproteases, thiol proteases, exopeptidases and the like. Of these, serine and cysteine protease inhibitors are of particular interest, with metalloprotease and aspartic inhibitors also being significant. Non-limiting examples of serine protease inhibitors include antipain, aprotinin, chymostatin, elastatinal, phenylmethylsulfonyl fluoride (PMSF), APMSF, TLCK, TPCK, leupeptin and soybean trypsin inhibitor. Inhibitors of cysteine proteases include, for example, IAA (indoleacetic acid) and E-64. Suitable examples of aspartic protease inhibitors include pepstatin and VdLPFFVdL. Non-limiting examples of inhibitors of metalloproteases include EDTA, as well as 1, 10-phenanthroline and phosphoramodon. Inhibitors of exopeptidases include, for example, amastatin, bestatin, diprotin A and diprotin B. Additional suitable examples of protease inhibitors include alpha-2-macroglobulin, soybean or lima bean trypsin inhibitor, pancreatic protease inhibitor, egg white ovostatin and egg white cystatin.

The stabilizing agent may be in any suitable form including, but not limited to, a solution, suspension or other liquid, a pellet, a tablet, a capsule, a spray-dried material, a freeze-dried material, a powder, a particle, a gel, crystals, substrate-bound additive, buffered matrix, or a lyophilized material. Because the half-life of many inhibitors is short, the stabilizing agent is preferably introduced into the collection device in such a form so as to optimize the shelf life of the inhibitor. Lyophilization appears to be particularly useful in that it provides good stability and also allows subsequent sterilization, both of which are key from a standpoint of automation, standardization, and clinical implementation.

The stabilizing agent may be located on any surface of the collection device. The stabilizing agent may also be located on stoppers, seals for closing such devices or on mechanical, component surfaces and sub-surfaces, or other, inserts placed within such devices. Preferably, the stabilizing agent is located anywhere along at least one interior wall of the collection device or anywhere within the reservoir portion. In addition, some phosphatase inhibitors may exhibit light sensitivity. Thus, it may be desirable to protect the agent from light. For such inhibitors, use of an opaque tube, *e*.*g*., an amber-colored tube with or without observation window, would be advantageous (Kirk, Scott, consider window on amber tube for independent claim). Alternatively, placing the agent into a capsule that protects it from light exposure, *e*.*g*., in powdered form, and then placing the capsule into the tube would also address this issue. Capsulating the agent may also prevent other undesirable interactions between the agent and other elements in the container. Capsule materials that dissolve upon sample collection are well known in the art.

The stabilizing agent may be applied to the collection device by any number of methods. For example, the stabilizing agent may be spray dried, loosely dispensed or lyophilized over the surface of the interior wall of the collection device. Alternatively, the stabilizing agent, such as when in gel or liquid form, for example, may be positioned in the reservoir portion of the collection device. Additional methods for providing the collection device with the stabilizing agent are also possible. Typically, to dispose the desired amount of agent into a container, one reconstitutes a solid form of the agent and then dispenses the appropriate amount of liquid into the container. The liquid may be spray dried, disposed into the bottom of the container or subsequently lyophilized.

The quantity and location of the stabilizing agent are determined by several variables, including the mode of application, the specific stabilizing agent used, the internal volume and internal pressure of the collection device, and the volume of the biological sample drawn into the container.

The concentration of the stabilizing agent is sufficient to stabilize the protein and to inhibit or prevent protein degradation.

In addition to the stabilizing agent, the device of the present invention may also contain carrier media (*e*.*g*., water or alcohol), stabilizing media (*e*.*g*., polyvinylpyrollidone, trehalose mannitol, etc.) and/or one or more other additives for treating the biological sample. Suitable additives include, but are not limited to, phenol, phenol/chloroform mixtures, alcohols, aldehydes, ketones, organic acids, salts of organic acids, alkali metal salts of halides, organic chelating agents, fluorescent dyes, antibodies, binding agents, anticoagulants such as sodium citrate, heparin, potassium EDTA and the like, and any other reagent or combination of reagents normally used to treat biological samples for analysis. Other potential additives include antioxidants and reducing agents, which may help preserve protein confirmation, *e*.*g*., preserve sulfhydryl group couplings. It may also be advantageous to include a buffering agent or sugar compounds. Yet other additive groups or chemistries include those that enhance solubility of the preservative or stabilizer additive in the specimen matrix. Preferably, the carrier and additives do not degrade proteins. Where the stabilizing agent is in tablet form, pharmaceutical tablet disintegrating materials, which are known to those skilled in the art, may be included, if desired.

The methods include obtaining a biological sample and introducing the sample into the container containing the stabilizing agent. In preferred embodiments, the biological sample is withdrawn from the patient directly into the collection container without any intervening process steps. It has been found that collecting the biological sample directly from the patient, such as when collecting a whole blood sample, and introducing the sample directly into the container containing the stabilizing agent substantially reduces or prevents the modification, degradation and/or fragmentation of proteins that otherwise occurs when the sample is stored before combining it with the stabilizing agent. The method is useful both with open collection systems and with closed collection systems wherein the opening is closed by a closure means.

In a preferred embodiment, the collection device of the present invention is for drawing a whole blood sample directly from a patient for stabilizing the proteins immediately at the point of collection. The device may be an evacuated system for collecting blood. Alternatively, the device may be a partially-evacuated or a non-evacuated system for collecting blood. A suitable example of an evacuated system is a closed tube. A manual syringe draw is a suitable example of both a partially-evacuated and a non-evacuated system. Non-evacuated systems may also include automatic draw systems. Evacuated systems are particularly preferred.

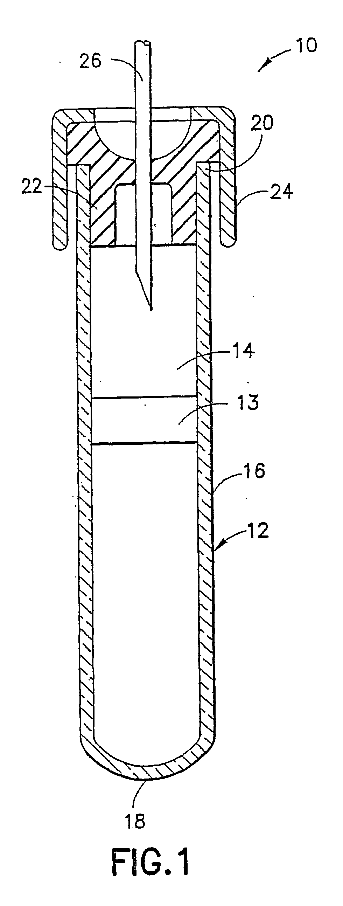

Referring to the drawings in which like reference characters refer to like parts throughout the several views thereof, FIG. 1 shows a typical blood collection device **10,** which includes a container **12** defining an internal chamber **14.** In the embodiment illustrated, container **12** is a hollow tube having a side wall **16,** a closed bottom end **18** and an open top end **20.** Optionally, a separating member **13** is provided within the container chamber **14.** Separating member **13** serves to assist in separating components of the sample, for example, by centrifugation. Container **12** is dimensioned for collecting a suitable volume of biological fluid, preferably blood. A closure means **22** for covering open end **20** to close container **12** is necessary where a sterile product is demanded. For conventional tubes, a screw cap is normally sufficient. For evacuated collection tubes, a tight-fitting, elastomeric plug is generally employed to contain the vacuum during the required storage periods. Preferably, closure **22** forms a seal capable of effectively closing container **12** and retaining a biological sample in chamber **14.** Closure **22** may be one of a variety of forms including, but not limited to, rubber closures, metallic seals, metal-banded rubber seals and seals of different polymers and designs. A protective shield **24** may overlie closure **22.** Container **12** also contains a stabilizing agent in accordance with the present invention.

Container **12** can be made of glass, plastic or other suitable materials. Preferably, container **12** is transparent. Non-limiting examples of suitable transparent thermoplastic materials for container **12** are polycarbonates, polyethylene, polypropylene and polyethyleneterephthalate. Plastic materials can be oxygen impermeable materials or may contain an oxygen impermeable or semi-permeable layer. Alternatively, container **12** can be made of a water and air permeable plastic material. The stabilizing agent may be provided to the container using any appropriate means. In one aspect, the stabilizing agent is in a liquid solution and is placed into the container. Subsequently, the solution may be lyophilized by methods that are known in the art such as, for example, freeze drying. For example, by freezing the solution and then slowly warming after freezing, while simultaneously applying a vacuum, a freeze-dried powder remains in the collection tube. An additive such as an excipient, for example, PVP or trehalose, may also be added to the stabilizing agent solution prior to freeze drying so that the resulting stabilizing agent is pelletized in the container. Vacuum drying may also be used after adding the stabilizing solution. In another aspect, the stabilizing agent is formed into a liquid or solid aerosol and sprayed onto one or more surfaces of the interior of the container.

The pressure in chamber **14** is selected to draw a predetermined volume of biological sample into chamber **14.** Preferably, closure **22** is made of a resilient material that is capable of maintaining the internal pressure differential between atmospheric pressure and a pressure less than atmospheric. Closure **22** is such that it can be pierced by a needle **26** or other cannula to introduce a biological sample into container **12** as known in the art. Preferably, closure **22** is resealable. Suitable materials for closure **22** include, for example, silicone rubber, natural rubber, styrene butadiene rubber, ethylene-propylene copolymers and polychloroprene.

Suitable examples of container **12** include single-wall and multi-layer tubes. A more specific example of a suitable container **12** is disclosed in U.S. Patent No. 5,860,937 to Cohen.

A useful manufacturing process for devices according to the present invention involves obtaining a collection container; adding at least one phosphatase inhibitor to the container; lyophilizing the at least one inhibitor; evacuating the container; and sterilizing the container. The at least one inhibitor may be dispensed into the container in solution form. After adding the inhibitor to the collection container, a separating member may be added to the container, if desired.

As noted, container **12** may also contain a gel, mechanical or other separating member (*e*.*g*., a filter mechanism). In such cases, the stabilizing agent may be spray dried and/or lyophilized on an exterior surface of the separation media. Container **12** may also be a collection device for blood plasma preparation. Such a collection device comprises, in addition to the stabilizing agent, an element for separating plasma from human or animal whole blood. The element for separating plasma from whole blood may be a separating member such as a gel formulation, a mechanical media or a filter mechanism. The gel is desirably a thixotropic polymeric gel formulation. The gel may be a homopolymer or a copolymer and may include silicone-based gels such as, for example, polysiloxanes, or organic hydrocarbon-based gels such as, for example, polyacrylics, polyesters, polyolefins, oxidized cis polybutadienes, polybutenes, blends of epoxidized soybean oil and chlorinated hydrocarbons, copolymers of diacids and propandiols, hydrogenated cyclopentadienes and copolymers of alpha-olefins with dialkylmaleates. The gel desirably isolates the plasma from the cells of the blood sample in the tube by serving as a density separation medium. An example of a suitable plasma preparation tube is disclosed in U.S. Patent No. 5,906,744 to Carroll et al., which is hereby incorporated by reference in its entirety. In this way, stabilization can be provided both before, during and after centrifugation to separate the plasma from the blood. In the case of a gel separating material, it may be desirable to provide physical/chemical separation between the stabilizing agent and the gel, *e*.*g*., use of a capsule as discussed above. For example, if portions of the agent are incorporated into or react with the gel, the effectiveness of the agent may be reduced. For the same reasons, where a mechanical separating element is used, the element is desirably substantially inert to the stabilizing agent, and this reflects a significant advantage of such a separator. Providing a separating element in plasma tubes, versus centrifuging without a separating element, is particularly advantageous. Specifically, because cell lysing releases proteases that degrade proteins of interest, the better the separation between the cells (*i*.*e*., the clotted blood) and the plasma, the better the stability of proteins in the plasma sample. Useful mechanical separators are found, for example, in U.S. Patents Nos. 6,516,953; 6,406,671; 6,409,528; and 6,497,325. Useful filter mechanisms are found, for example in U.S. Patents No. 6,506,167 by Ishimito et al. disclose a blood separating tube including an upstream tube separated by a filter from a downstream tube where the tubes are attachable to and detachable from each other and are evacuated. During blood collection, blood is removed from a patient through intravenous puncture and transferred into the upstream tube through blood pressure and negative pressure inside the tube. In accord with the disclosure, a pressure differential is supposed to be created between the upstream tube and the downstream tube as the blood contacts the filter between the two tubes. Thus centrifugation is not required in order to separate the whole blood. Several suggested filters include a membrane, glass fibers, filter paper with large pores having attached thereto anti-hemocyte antibodies, a filter impregnated with a cationic macromolecular substance to aggregate cells, and a laminated multi-layer filter.

Container **12** may also be a collection tube for centrifugally separating lymphocytes and monocytes from heavier phases of a sample of whole blood comprising, in addition to the stabilizing agent, a liquid density gradient medium and a means for preventing mixing of the liquid density gradient medium with a blood sample prior to centrifugation. An example of a suitable lymphocyte/monocyte collection tube is disclosed in U.S. Patent No. 5,053,134 to Luderer et al., which is hereby incorporated by reference in its entirety.

In another embodiment, a kit having at least two containers comprising one or more stabilizing agents is provided. For example, the kit may comprise a primary collection tube, *e*.*g*., a plasma separating tube having a separating element therein, and a secondary tube for testing, *e*.*g*., for pouring or otherwise dispensing the collected plasma into. Both would have stabilizing agent(s) therein, to ensure that the proteins of interest remained stable throughout. The tube may have stages whereby certain proteins are separated or depleted in one stage and stabilized in another. Optionally, the kit could include a tube-to-tube transfer device to prevent the need for pouring or other unsafe transfer practices, in which case the secondary tube would be at a reduced pressure to draw in the plasma. One using such a kit would collect a sample in the primary tube, centrifuge, transfer the sample of interest to the secondary testing tube, and perform the testing. The secondary testing tube could be of a variety of sizes, depending on the desired testing.

In another embodiment, the container is a tube with two open ends having closures thereon. Such a tube would allow one to sample, *e*.*g*., for a plasma separating tube with a separating element therein, either the plasma sample or the clot sample.

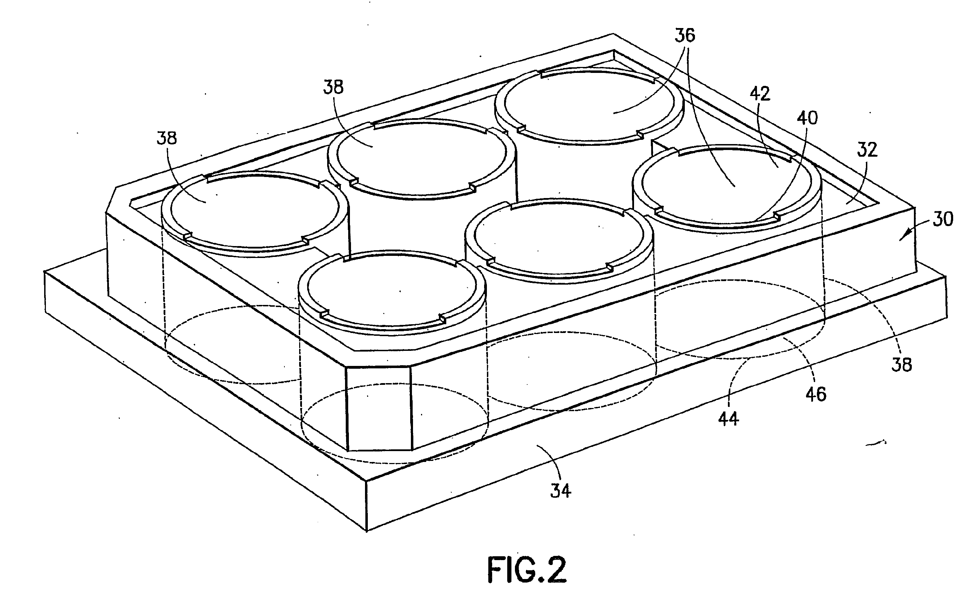

In yet another embodiment, the collection device of the present invention comprises a test plate such as, for example, a single- or multi-well plate, a microtiter plate, a tissue culture plate or the like. A typical test plate generally comprises one or more wells, which are preferably cylindrical. As shown in FIG. 2, a test plate **30** includes an upper surface **32** and a lower surface **34.** Test plate **30** further includes a number of wells **36** each comprising a sidewall **38** extending from upper surface **32** of the plate to lower surface **34** of the plate. Each well comprises a top portion **40** and a bottom portion **44.** Top portion **40** comprises an open end **42** that extends to bottom portion **44,** which comprises a closed end **46.** Bottom portion **44** may be flat, conical (pointed) or rounded. The capacity of each well **36** typically ranges from several milliliters (ml) to less than about 0.5 ml. Wells **36** may each accommodate therein a stabilizing agent according to the present invention.

The number of wells **36** in test plate **30** is not critical. There may be any number of wells, although six-, twelve-, twenty-four-, forty-eight- and ninety-six-well test plates are commonly known and available. In FIG. 2, a six-well test plate is illustrated, merely for exemplary purposes, and the invention is not dependent upon the number of wells. Most standard multi-well plates have the wells arranged in orthogonal rows and columns so as to be able to clearly identify the individual wells being used. Of course, the arrangement of the wells in test plate **30** is not an essential limitation of the present invention because any arrangement of wells is contemplated by the invention.

Plate **30** may be formed from thermoplastic materials by vacuum forming, sheet molding, injection molding or other similar techniques. Suitable thermoplastic materials include, but are not limited to, polystyrene, polyvinylchloride, polycarbonate, polyethyleneterephthalate and the like. Preferably, plate **30** is transparent.

Surrounding the wells and forming the outside border of test plate **30** are sidewalls **38.** In the present embodiment, test plate **30** has six (6) sidewalls. Well known test plates are rectangle or quadrilaterally shaped, although for purposes of the present invention the plate may be fabricated in any practical configuration. Examples of suitable test plates containing a plurality of wells are disclosed in U.S. Patent No. 5,882,922 to Tyndorf et al., U.S. Patent No. 5,801,055 to Henderson and U.S. Patent No. 5,681,743 to Brian et al.,.

In yet another embodiment, the collection device according to the present invention may be a sample collection assembly for the collection, transport and dispensing of biological samples. The collection assembly generally includes a plurality of sample wells for collecting individual biological samples. The sample wells are supported in a sample tray in a spaced-apart orientation. The sample tray may be supported within a case that encloses the sample tray and allows the safe and efficient transport of the sample wells. The sample tray is movably accommodated within the case for movement between a first position enclosing the plurality of sample wells, to a second position rendering exteriorly accessible one of the sample wells so that the sample can be manually dispensed from the tray.

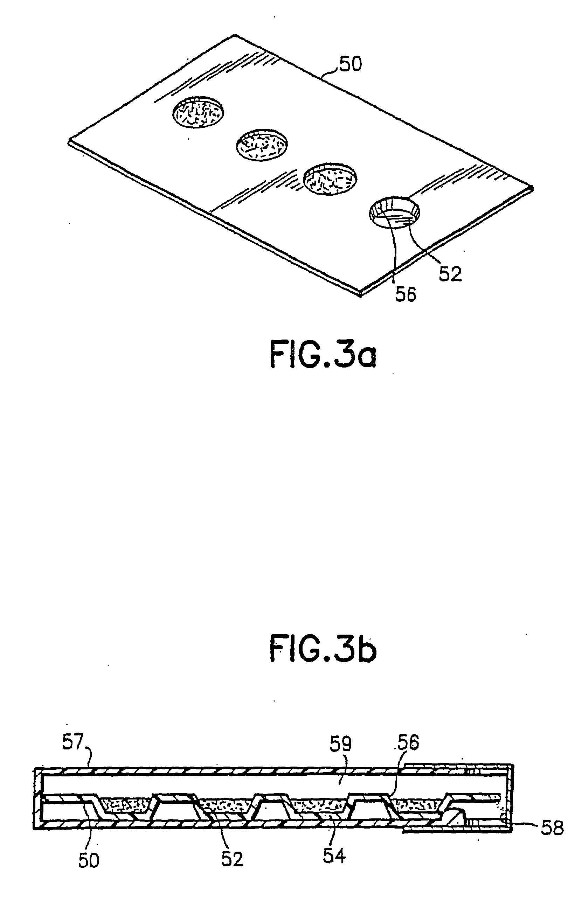

As shown in FIGS. 3a and 3b, sample tray **50** includes a plurality of longitudinally spaced depressions forming specimen collection wells **52**. Sample tray **50** may be formed of a suitably deformable plastic material. Wells **52** have a bottom **54** and an open end **56.** It is contemplated that the sample wells may be in the shape of open ended cup-like members. Wells **52** are constructed to have sufficient depth so as to retain a suitable volume of a biological sample. Wells **52** may each accommodate therein a stabilizing agent according to the present invention. While tray **50** of the present invention is shown having a single row of wells **52** formed therein, the present invention contemplates that the wells may be provided in any number or any array desirable for a particular testing situation. The sample collection assembly may include a sample collection case **57.** Upon collection of a biological sample within wells **52,** sample tray **50** may be inserted into the open end **58** of sample collection case **57** and then within the interior **59** of sample collection case **57** until all of wells **52** are enclosed therein. A suitable sample collection assembly is disclosed in U.S. Patent No. 6,357,583 B1 to Rainen.

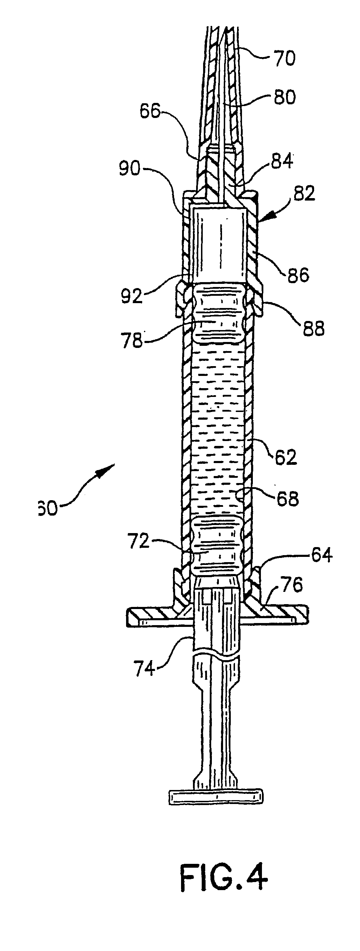

According to another embodiment of the present invention, as depicted in FIG. 4, the collection device comprises a syringe and, more preferably, a syringe pre-filled with a stabilizing agent in accordance with the present invention. A typical syringe comprises a generally cylindrical barrel having opposed proximal and distal ends with at least one chamber formed between the ends for receiving a substance such as a biological sample. A plunger is typically sealably disposed within the barrel and movable with respect thereto, and sealing means may be sealably disposed approximate to the distal end of the barrel. Referring now to FIG. 4, there is shown a syringe **60,** which includes an elongate barrel or cylinder **62** having an open, proximal end **64** and a distal end **66,** with at least one hollow chamber **68** formed between the proximal and distal ends for receiving a biological sample. In the embodiment illustrated, distal end **66** includes a needle guard **70.** The needle guard keeps the syringe, as well as the needle, sterile during storage.

The barrel of the syringe includes a stabilizing agent. Preferably, the barrel of the syringe is pre-filled with the stabilizing agent. Pre-filled syringes, as the term is known in the art, are syringes that are filled by the manufacturer and shipped to the health care provider ready for use.

A plunger **72** may be situated at open, proximal end **64.** Plunger **72** can be moved by means of a plunger rod **74,** which is secured to the plunger, for example, by screwing. At the same end where the plunger is situated, the barrel may have a fingergrip **76,** which is secured to the barrel according to the so-called snap-cap principle. Fingergrip **76** preferably consists of slightly resilient material, for example plastics. In another embodiment (not shown), the fingergrip is a flange-like part of the barrel projecting radially outwards. Of course, other constructions known to those skilled in the art are possible.

A stopper **78,** which closes the barrel, may be situated in the end of the barrel remote from the plunger. The plunger and the stopper are preferably manufactured from an elastic material and, most preferably, from rubber of a pharmaceutical quality.

In the embodiment illustrated, an injection needle **80** is secured to the barrel by means of a needle holder **82.** The needle holder has a neck **84,** which holds the needle, a shaft **86** and a collar **88.** The needle holder is preferably manufactured from slightly resilient material that has resistance to deformation such as, for example, plastics, and is secured to the end of the barrel by means of a snap-cap construction. In the alternative, the needle holder may be secured to the barrel by means of a screwed or adhesive connection or, when the barrel also comprises a collar, by means of a clamping ring. In the latter embodiment, the needle holder may also be flanged around a collar of the barrel.

Although the syringe barrel illustrated in this embodiment includes a locking Luer-type collar **88,** it is within the purview of the present invention to include syringe barrels without a collar, syringe barrels having an eccentrically positioned nozzle and various other nozzle-like structures adapted to accept, either permanently or removably, a needle cannula or needle cannula assembly. It is only required that there is an aperture on the distal end of the syringe barrel in fluid communication with the interior of the syringe barrel.

One or more slots **90** may be recessed in the inner wall of shaft **86** and the rear face of neck **84.** The slot or slots extend into the rear end of the cannula. In cross-section, the slots may be parts of a circle, but other shapes are also possible, provided the size is such that sufficient injection liquid can be readily passed through; this is achieved if the diameter of the slot or the overall cross-section of the slots is at least as large as that of the cannula. Shaft **86** of needle holder **82** is constructed so that when stopper **78** slides axially forward, it is received, with friction, by the shaft; therefore, apart from slots **90** recessed in the shaft, the inside diameter of the shaft is approximately as large as that of barrel **62.** Shaft **86** of needle holder **82** is slightly longer than stopper **78** so that the part **92** of the slot(s) adjoining the barrel is free when the stopper is moved forward against the rear wall of the neck of the needle holder. If desired, needle guard **70** may be constructed to also serve as a plunger rod. In that case, prior to use of the syringe, the needle guard is removed from the needle and secured at the other end of the syringe to the plunger.

Generally, a syringe comprising a needle protector has a safety member, which indicates whether the needle protector has previously been removed. Such a safety member in the form of a cap is described in, for example, U.S. Patent No. 3,995,630.

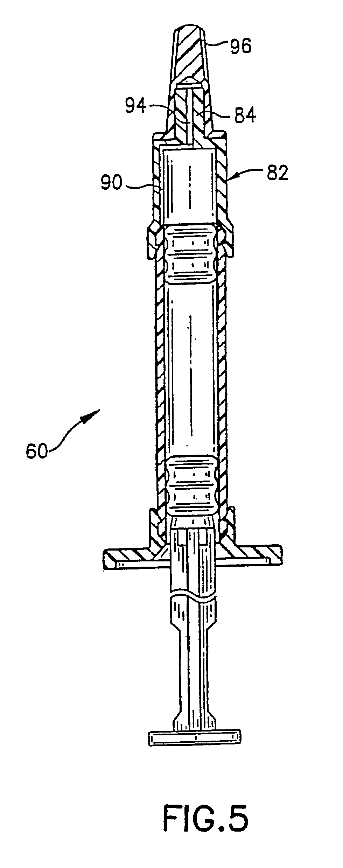

In further embodiments, the syringe is not stored with a needle in position, *i*.*e*., it is a needleless syringe as known in the art. This is illustrated in FIG. 5. With such a syringe, before use, the needle is positioned on neck **84** of needle holder **82** by means of a needle hub. A so-called Luer cone is preferably used for this connection. In this embodiment, aperture **94** in the neck of the needle holder is closed on the outside by a protective cap **96,** which ensures the sterility of the syringe as well as the needle holder. Slot **90** recessed in the needle holder projects into the end of the neck aperture.

An example of a suitable syringe is disclosed in U.S. Patent No. 6,027,481 to Barrelle et al., which is hereby incorporated by reference in its entirety. Other examples of suitable syringes are disclosed in, for example, U.S. Patent No. 4,964,866 to Szwarc, U.S. Patent No. 4,986,818 to Imbert et al., U.S. Patent No. 5,607,400 to Thibault et al. and U.S. Patent No. 6,263,641 B1 to Odell et al..

In a further embodiment, the collection device comprises a catheter. As known in the art, catheters are commonly employed when a patient requires repeated doses of medication or other substances. A catheter permits repeated and continuous administration of medication directly into a patient's blood stream, or other region of the body, without repeated injections. Typically, catheters have a hollow tubular lumen, a proximal end and a distal end. The distal end of the catheter, which may be open or closed, is inserted into the vein or artery of a patient.

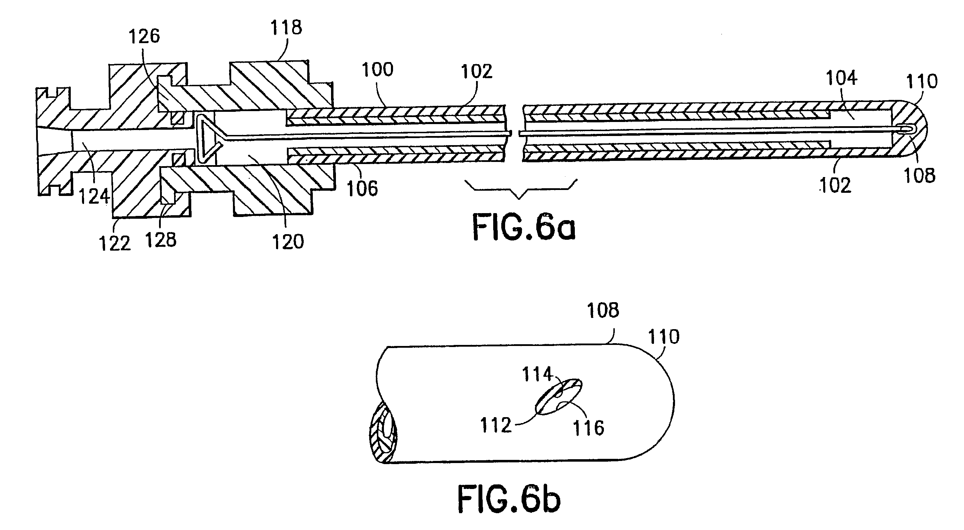

FIG. 6a illustrates an exemplary catheter assembly that includes a flexible catheter **100** having a cylindrical side wall **102** describing a lumen **104** therethrough, a proximal end **106** and a closed distal end **108** which, in this illustrated embodiment, has a rounded exterior surface **110** to facilitate insertion of the catheter into the patient. As illustrated in FIG. 6b, catheter **100** includes a slit **112** through side wall **102** adjacent to distal end **108** and is defined by two opposed faces **114** and **116** formed in the side wall. Catheter **100** includes a stabilizing agent according to the present invention, preferably in the lumen of the catheter.

The proximal end of the catheter is connected to a catheter housing **118** having a conduit **120** therethrough. Conduit **120** in the catheter housing and lumen **104** in the catheter are in fluid communication. A valve control knob **122** having a passageway **124** therethrough is rotatably connected to catheter housing **118** so that passageway **124** is in fluid communication with conduit **120.** Valve control knob **122** and catheter housing **118** are held together by virtue of proximal flange **126** on the catheter housing which, engages rotational groove **128** in the valve control knob. This structure allows the valve control knob to rotate with respect to the catheter housing but keeps the two elements from coming apart. An example of a suitable catheter is disclosed in U.S. Patent No. 4,737,152 to Alchas, which is hereby incorporated by reference in its entirety.

In yet a further embodiment, the collection device comprises a pipette. In laboratory settings, it is well known to use a pipette to extract a certain volume of a biological fluid from one container and to transport and dispense some or all of the extracted volume into another container. Typically, pipettes are generally hollow tubular members that are used by applying suction at an open upper end, or mouthpiece, in order to extract or aspirate a quantity of fluid medium into the hollow tube. A pressure differential maintained by closing the mouthpiece opening retains the fluid within the pipette allowing transport of the fluid medium to another container. Selective opening of the mouthpiece allows a quantity of the fluid medium contained in the pipette to be dispensed. A certain degree of accuracy in the amount of fluid dispensed is provided by the tapered end portions by reducing the amount of fluid lost due to dripping.

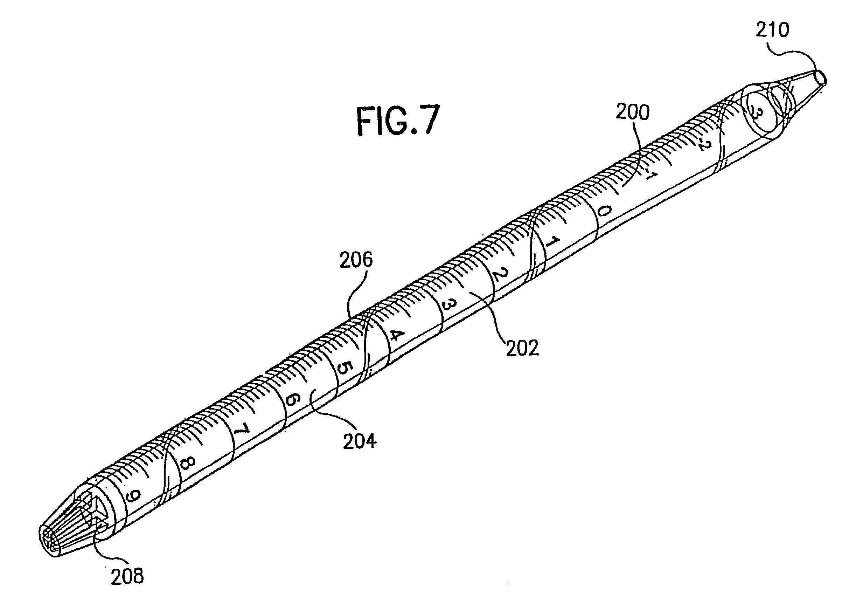

Referring now to FIG. 7, an exemplary pipette **200** is shown. Pipette **200** is generally an elongate tubular member defined by a tubular wall **202** of generally uniform thickness. Within tubular wall **202,** a pipette interior **204** is defined for accommodating a given volume of fluid medium, for example, a biological sample. Pipette **200** includes an elongate generally cylindrical main body portion **206** that is coextensive with interior **204.** Pipette body **206** may be pre-filled with a stabilizing agent according to the present invention.

In order to aspirate and dispense a biological fluid, pipette **200** includes a dispensing portion **208** at one end of body **206** and a mouthpiece **210** at the other end. Both dispensing portion **208** and mouthpiece **210** are in communication with interior **204** of pipette **200** so as to permit aspirating and dispersing of the fluid through dispensing portion **208** by creating a selective pressure differential within interior **204** of pipette **200** using mouthpiece **210.** Such a pressure differential can be created manually by opening and closing mouthpiece **210** or may be created by use of mechanical pipette aids.

Pipette **200** may be constructed of glass or a thermoplastic material such as polycarbonate, polyethylene, polyester, polystyrene, polypropylene, polysulfone, polyurethane, ethylene vinyl acetate or the like. Thermoplastic pipettes have largely replaced glass pipettes for many uses. The material of pipette **200** may be transparent, translucent or opaque.

Examples of suitable pipettes are disclosed in, for example, U.S. Patent No. 6,280,689 B1 to Stevens and U.S. Patent No. 6,343,717 B1 to Zhang et al.,.

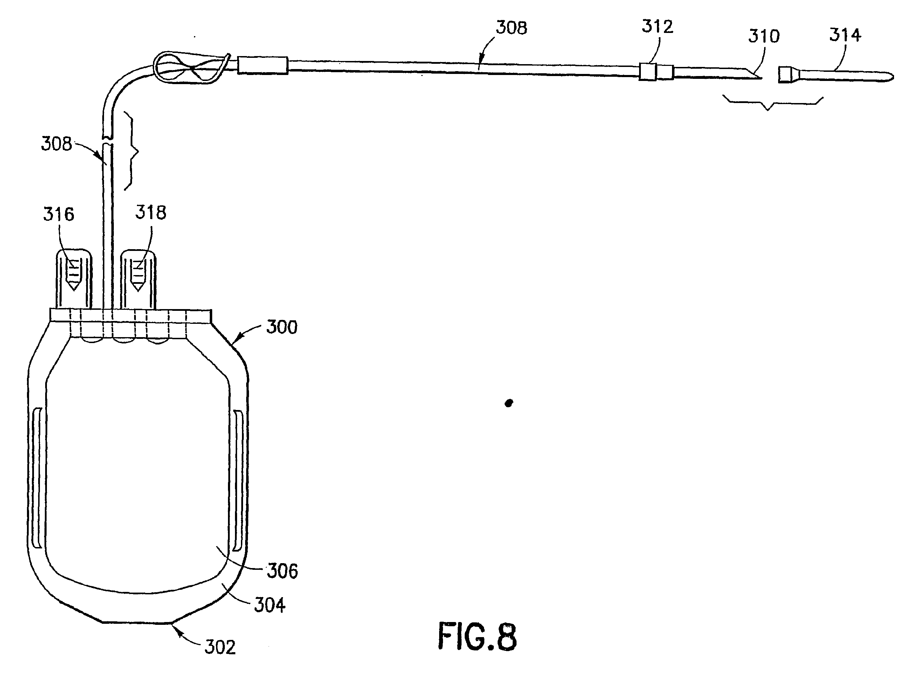

The collection device may also comprise a collection bag suitable for holding a biological sample such as, for example, a blood collecting bag, a blood plasma bag, a buffy coat bag, a platelet bag or the like. For ease of description, a blood collecting bag will now be described with reference to FIG. 8.

FIG. 8 illustrates a blood collecting bag **300** for accommodating collected blood. Blood collecting bag **300** has a body **302** formed by superposing a pair of identically cut pieces of a sheet material made of a resin, which will be more specifically described hereinafter, and possessed of flexibility and fusing (*i*.*e*., heat fusion, high frequency fusion or the like) or adhesively joining to each other the periphery of the sealing portion **304** of each of the pieces of sheet material. A blood-accommodating portion **306** accommodating collected blood is formed at an inner portion surrounded with sealing portion **304** of body 302. Blood collecting bag **300** preferably contains a stabilizing agent in accordance with the present invention.

One end of the flexible tube **308** communicating with blood-accommodating portion 306 is connected with body **302** at an upper portion thereof. A blood collecting needle **310** is installed at the other end of flexible tube **308** through a hub **312.** A cap **314,** which is to cover blood collecting needle **310,** may be installed on hub **312.** Two openings **316** and **318,** each sealed with a peel tab, may be formed at an upper portion of body **302** such that they can be opened.

The composition, characteristics and the like of the material of the sheets composing body **302** of blood collecting bag **300** are not limited to specified ones. In this case, as the sheet material composing blood collecting bag **300,** soft polyvinyl chloride or materials containing the soft polyvinyl chloride as their main component is preferably used. For example, a copolymer containing the soft polyvinyl chloride as its main component and a small amount of macromolecular material, a polymer blend, a polymer alloy and the like can be used. As the plasticizer for the soft polyvinyl chloride, dioctylphthalate (DEHP, di(2-ethylhexyl)phthalate) and (DnDP, di(n-decyl)phthalate) can be preferably used. The content of such a plasticizer in the polyvinyl chloride is preferable to be in the approximate range of 30 to 70 parts by weight, based on 100 parts by weight of polyvinyl chloride.

The other substances that are effectively usable for the sheet material of blood collection bag **300** are polyolefins, *i*.*e*., the products of homopolymerization or copolymerization of such olefins or diolefins as ethylene, propylene, butadiene and isoprene. Typical examples include polyethylene, polypropylene, ethylene vinyl acetate copolymer (EVA), polymer blends formed between EVA and various thermoplastic elastomers and arbitrary combinations thereof Such polyesters as polyethylene terephthalate (PET), polybutylene terephthalate (PBT), poly-1,4-cyclohexane dimethyl terephthalate (PCHT) and polyvinylidene chloride are also usable.

In yet another embodiment, the collection device of the present invention may be a laboratory vessel that contains the stabilizing agent. Particular vessels that can be used in accordance with the present invention include, for example, vials, flasks, spinner flasks, roller bottles, microscope slides, microscope slide assemblies, sample chambers for analytical devices, tapes, laminates, arrays, tubing and the like. Laboratory vessels according to the present invention have at least one operational surface. Many vessels according to the invention have at least one interior wall, which defines a reservoir portion for containing the biological sample, and at least one opening in communication with the reservoir portion.

Plastic or glass is often used to manufacture the laboratory vessels. Some preferred materials used to manufacture laboratory vessels include polypropylene, polyethylene, polyethyleneterephthalate, polystyrene, polycarbonate and cellulosics. Because polypropylene is inexpensive, it is a particularly preferred material for laboratory vessels used for handling and transporting minute and precise amounts of biological sample.

Examples of other suitable materials for the laboratory vessels of the present invention include polyolefins, polyamides, polyesters, silicones, polyurethanes, epoxies, acrylics, polyacrylates, polyesters, polysulfones, polymethacrylates, PEEK, polyimide and fluoropolymers. Glass products including silica glass are also used to manufacture laboratory vessels.

While various embodiments have been chosen to demonstrate the invention, it will be understood by those skilled in the art that various modifications and additions can be made without departing from the scope of the invention as defined in the appended claims.

## Claims

1. An apparatus for collecting a biological sample, comprising:
a container having a reservoir portion for receiving the sample; and
at least one protein stabilizing agent comprising at least one phosphatase inhibitor in the reservoir of the container.

2. The apparatus of claim 1, wherein the container is selected from the group consisting of tubes, closed system blood collection devices, collection bags, syringes, pre-filled syringes, catheters, microtiter plates, multi-well collection devices, flasks, spinner flasks, roller bottles, vials, pipettes, pipette tips and tissue and other biological sample collection containers.

3. The apparatus of claim 1, wherein the container is a tube having a first end and a second end.

4. The apparatus of claim 3, further comprising a separating member disposed in the container.

5. The apparatus of claim 4, wherein the separating member is a mechanical separating element.

6. The apparatus of claim 5, wherein the mechanical separating element is at least partially coated with the at least one stabilizing agent.

7. The apparatus of claim 5, wherein the mechanical separating element is substantially inert with respect to the stabilizing agent.

8. The apparatus of claim 4, wherein the separating member is a gel.

9. The apparatus of claim 8, wherein the gel separating member is physically separated from the stabilizing agent.

10. The apparatus of claim 1, wherein the protein stabilizing agent is in a form selected from the group consisting of a solution, suspension or other liquid, a pellet, a tablet, a capsule, a spray-dried material, a freeze-dried material, a powder, a particle, a gel, crystals or a lyophilized material.

11. The apparatus of claim 10, wherein the protein stabilizing agent is lyophilized.

12. The apparatus of claim 1, wherein the at least one phosphatase inhibitor, inhibits at least one phosphatase selected from the group consisting of serine phosphatase, threonine phosphatase, and tyrosine phosphatase.

13. The apparatus of claim 1, wherein the stabilizing agent further comprises at least one protease inhibitor.

14. The apparatus of claim 1, wherein the stabilizing agent comprises a phosphatase inhibitor cocktail.

15. The apparatus of claim 1, wherein the stabilizing agent comprises more than two phosphatase inhibitors.

16. The apparatus of claim 1, further comprising a carrier media.

17. The apparatus of claim 1, further comprising a stabilizing media.

18. The apparatus of claim 17, wherein the stabilizing media is trehalose.

19. The apparatus of claim 1, further comprising at least one antioxidant.

20. The apparatus of claim 1, further comprising at least one reducing agent.

21. The apparatus of claim 1, further comprising at least one buffering agent.

22. The apparatus of claim 3, wherein the first end is an open end, the second end is a closed end, and further comprising a closure means for sealing the first open end.

23. The apparatus of claim 3, wherein the tube is partially evacuated.

24. The apparatus of claim 3, wherein the first end is an open end, the second end is an open end, and further comprising a first closure means for sealing the first open end and a second closure means for sealing the second open end.

25. The apparatus of claim 3, wherein the protein stabilizing agent is lyophilized.

26. The apparatus of claim 25, wherein the protein stabilizing agent comprises more than two phosphatase inhibitors.

27. The apparatus of claim 25, wherein the tube further comprises an anticoagulant.

28. The apparatus of claim 27, wherein the anticoagulant is spray-dried onto at least a portion of an interior wall.

29. The apparatus of claim 27, wherein the anticoagulant comprises a salt of EDTA.

30. The apparatus of claim 27, wherein the anticoagulant comprises heparin.

## Patentansprüche

1. Vorrichtung zur Aufnahme einer biologischen Probe, umfassend:
einen Behälter mit einem Reservoirteil zur Aufnahme der Probe; und
wenigstens ein Protein-Stabilisierungsmittel, das wenigstens einen Phosphatase-Inhibitor in dem Reservoir des Behälters umfasst.

2. Vorrichtung gemäß Anspruch 1, wobei der Behälter aus der Gruppe ausgewählt ist, die aus Röhrchen, Blutentnahmevorrichtungen in Form geschlossener Systeme, Sammelbeuteln, Spritzen, vorgefüllten Spritzen, Kathetern, Mikrotiterplatten, Multiwell-Sammelvorrichtungen, Kolben, Spinnerflaschen, Rollerflaschen, Stechampullen, Pipetten, Pipettenspitzen und Sammelbehältern für Gewebe und andere biologische Proben besteht.

3. Vorrichtung gemäß Anspruch 1, wobei der Behälter ein Röhrchen mit einem ersten Ende und einem zweiten Ende ist.

4. Vorrichtung gemäß Anspruch 3, die weiterhin ein in dem Behälter befindliches Trennelement umfasst.

5. Vorrichtung gemäß Anspruch 4, wobei das Trennelement ein mechanisches Trennelement ist.

6. Vorrichtung gemäß Anspruch 5, wobei das mechanische Trennelement wenigstens teilweise mit dem wenigstens einen Stabilisierungsmittel beschichtet ist.

7. Vorrichtung gemäß Anspruch 5, wobei das mechanische Trennelement bezüglich des Stabilisierungsmittels im Wesentlichen inert ist.

8. Vorrichtung gemäß Anspruch 4, wobei das Trennelement ein Gel ist.

9. Vorrichtung gemäß Anspruch 8, wobei das Gel-Trennelement physikalisch von dem Stabilisierungsmittel getrennt ist.

10. Vorrichtung gemäß Anspruch 1, wobei das Protein-Stabilisierungsmittel in einer Form vorliegt, die aus der Gruppe ausgewählt ist, die aus einer Lösung, Suspension oder anderen Flüssigkeiten, einem Sediment, einer Tablette, einer Kapsel, einem sprühgetrockneten Material, einem gefriergetrockneten Material, einem Pulver, einem Teilchen, einem Gel, Kristallen oder einem lyophilisierten Material besteht.

11. Vorrichtung gemäß Anspruch 10, wobei das Protein-Stabilisierungsmittel lyophilisiert ist.

12. Vorrichtung gemäß Anspruch 1, wobei der wenigstens eine Phosphatase-Inhibitor wenigstens eine Phosphatase hemmt, die aus der Gruppe ausgewählt ist, die aus Serin-Phosphatase, Threonin-Phosphatase und Tyrosin-Phosphatase besteht.

13. Vorrichtung gemäß Anspruch 1, wobei das Stabilisierungsmittel weiterhin wenigstens einen Protease-Inhibitor umfasst.

14. Vorrichtung gemäß Anspruch 1, wobei das Stabilisierungsmittel ein Phosphatase-Inhibitor-Cocktail umfasst.

15. Vorrichtung gemäß Anspruch 1, wobei das Stabilisierungsmittel mehr als zwei Phosphatase-Inhibitoren umfasst.

16. Vorrichtung gemäß Anspruch 1, die weiterhin ein Trägermedium umfasst.

17. Vorrichtung gemäß Anspruch 1, die weiterhin ein Stabilisierungsmedium umfasst.

18. Vorrichtung gemäß Anspruch 17, die es sich bei dem Stabilisierungsmedium um Trehalose handelt.

19. Vorrichtung gemäß Anspruch 1, die weiterhin wenigstens ein Antioxidans umfasst.

20. Vorrichtung gemäß Anspruch 1, die weiterhin wenigstens ein Reduktionsmittel umfasst.

21. Vorrichtung gemäß Anspruch 1, die weiterhin wenigstens ein Puffermittel umfasst.

22. Vorrichtung gemäß Anspruch 3, wobei das erste Ende ein offenes Ende ist, das zweite Ende ein geschlossenes Ende ist und die Vorrichtung weiterhin ein Verschlussmittel zum Versiegeln des ersten, offenen Endes umfasst.

23. Vorrichtung gemäß Anspruch 3, wobei das Röhrchen teilweise evakuiert ist.

24. Vorrichtung gemäß Anspruch 3, wobei das erste Ende ein offenes Ende ist, das zweite Ende ein offenes Ende ist und die Vorrichtung weiterhin ein erstes Verschlussmittel zum Versiegeln des ersten offenen Endes und ein zweites Verschlussmittel zum Versiegeln des zweiten offenen Endes umfasst.

25. Vorrichtung gemäß Anspruch 3, wobei das Protein-Stabilisierungsmittel lyophilisiert ist.

26. Vorrichtung gemäß Anspruch 25, wobei das Protein-Stabilisierungsmittel mehr als zwei Phosphatase-Inhibitoren umfasst.

27. Vorrichtung gemäß Anspruch 25, wobei das Röhrchen weiterhin ein Antikoagulans umfasst.

28. Vorrichtung gemäß Anspruch 27, wobei das Antikoagulans auf wenigstens einen Teil einer Innenwand sprühgetrocknet ist.

29. Vorrichtung gemäß Anspruch 27, wobei das Antikoagulans ein Salz von EDTA umfasst.

30. Vorrichtung gemäß Anspruch 27, wobei das Antikoagulans Heparin umfasst.

## Revendications

1. Dispositif pour recueillir un échantillon biologique, comprenant:
un récipient ayant une partie réservoir pour recevoir l'échantillon; et
au moins un agent stabilisant les protéines comprenant au moins un inhibiteur de phosphatases.

2. Dispositif selon la revendication 1, dans lequel le récipient est choisi dans le groupe consistant en tubes, dispositifs de prélèvement de sang en système fermé, poches de recueil, seringues, seringues préremplies, cathéters, plaques de microtitration, dispositifs de recueil multipuits, flacons, flacons rotatifs, bouteilles pour agitateur rotatif, flacons-ampoules, pipettes, embouts de pipette et récipients pour prélèvement de tissus et d'autres échantillons biologiques.

3. Dispositif selon la revendication 1, dans lequel le récipient est un tube ayant une première extrémité et une seconde extrémité.

4. Dispositif selon la revendication 3, comprenant en outre un élément de séparation disposé dans le récipient.

5. Dispositif selon la revendication 4, dans lequel l'élément de séparation est un élément de séparation mécanique.

6. Dispositif selon la revendication 5, dans lequel l'élément de séparation mécanique est au moins partiellement revêtu de l'au moins un agent stabilisant.

7. Dispositif selon la revendication 5, dans lequel l'élément de séparation mécanique est sensiblement inerte par rapport à l'agent stabilisant.

8. Dispositif selon la revendication 4, dans lequel l'élément de séparation est un gel.

9. Dispositif selon la revendication 8, dans lequel l'élément de séparation sous forme de gel est séparé physiquement de l'agent stabilisant.

10. Dispositif selon la revendication 1, dans lequel l'agent stabilisant les protéines est sous une forme choisie dans le groupe consistant en une solution, suspension ou autres liquides, un culot, un comprimé, une capsule, un matériau séché par pulvérisation, un matériau séché par lyophilisation, un poudre, une particule, un gel, des cristaux ou un matériau lyophilisé.

11. Dispositif selon la revendication 10, dans lequel l'agent stabilisant les protéines est lyophilisé.

12. Dispositif selon la revendication 1, dans lequel ledit au moins un inhibiteur de phosphatases inhibe au moins une phosphatase choisie dans le groupe consistant en la sérine phosphatase, la thréonine phosphatase et la tyrosine phosphatase.

13. Dispositif selon la revendication 1, dans lequel l'agent stabilisant comprend en outre au moins un inhibiteur de protéase.

14. Dispositif selon la revendication 1, dans lequel l'agent stabilisant comprend un cocktail d'inhibiteurs de phosphatases.

15. Dispositif selon la revendication 1, dans lequel l'agent stabilisant comprend plus de deux inhibiteurs de phosphatases.

16. Dispositif selon la revendication 1, comprenant en outre un milieu support.

17. Dispositif selon la revendication 1, comprenant en outre un milieu stabilisant.

18. Dispositif selon la revendication 17, dans lequel le milieu stabilisant est le tréhalose.

19. Dispositif selon la revendication 1, comprenant en outre au moins un antioxydant.

20. Dispositif selon la revendication 1, comprenant en outre au moins un agent réducteur.

21. Dispositif selon la revendication 1, comprenant en outre au moins un agent tampon.

22. Dispositif selon la revendication 3, dans lequel la première extrémité est une extrémité ouverte, la seconde extrémité est une extrémité fermée, et le dispositif comprend en outre un moyen de fermeture pour fermer hermétiquement la première extrémité ouverte.

23. Dispositif selon la revendication 3, dans lequel le tube est évacué partiellement.

24. Dispositif selon la revendication 3, dans lequel la première extrémité est une extrémité ouverte, la seconde extrémité est une extrémité ouverte, et le dispositif comprend en outre un premier moyen de fermeture pour fermer hermétiquement la première extrémité ouverte et un second moyen de fermeture pour fermer hermétiquement la seconde extrémité ouverte.

25. Dispositif selon la revendication 3, dans lequel l'agent stabilisant les protéines est lyophilisé.

26. Dispositif selon la revendication 25, dans lequel l'agent stabilisant les protéines comprend plus de deux inhibiteurs de phosphatases.

27. Dispositif selon la revendication 25, dans lequel le tube comprend en outre un anticoagulant.

28. Dispositif selon la revendication 27, dans lequel l'anticoagulant est appliqué par séchage par pulvérisation sur au moins une partie de la paroi intérieure.

29. Dispositif selon la revendication 27, dans lequel l'anticoagulant comprend un sel de l'EDTA.

30. Dispositif selon la revendication 27, dans lequel l'anticoagulant comprend l'héparine.
